# EUROPÄISCHE PATENTSCHRIFT

(11) **EP 3 115 075 B1**
(45) Veröffentlichungstag und Bekanntmachung des Hinweises auf die Patenterteilung: **23.12.2020**
(21) Anmeldenummer: 16001486.6
(22) Anmeldetag: 05.07.2016
(51) Int. Cl.: A61M 16/00, A61B 5/029

(54) **VORRICHTUNG ZUR VERÄNDERUNG DES ATEMGAS-DRUCKES IN ABHÄNGIGKEIT VON EINEM MASS DER HERZLEISTUNG**
DEVICE FOR VARYING THE BREATH GAS PRESSURE DEPENDING ON A MEASURE OF CARDIAC OUTPUT
DISPOSITIF DESTINE A MODIFIER LA PRESSION DE GAZ RESPIRATOIRE EN FONCTION D'UNE MASSE DE LA PERFORMANCE CARDIAQUE

(30) Priorität: 07.07.2015 DE 102015008548; 07.07.2015 DE 102015008550
(43) Veröffentlichungstag der Anmeldung: 11.01.2017
(73) Patentinhaber: Löwenstein Medical Technology S.A., 2557 Luxembourg (LU)
(72) Erfinder: Schwaibold, Matthias, 76228 Karlsruhw (DE); Dömer, Benno, 76275 Ettlingen (DE); Alshut, Rüdiger, 76131 Karlsruhe (DE)
(74) Vertreter: Marx, Thomas

(56) Entgegenhaltungen:
- EP-A1- 2 923 720
- EP-A2- 2 216 063
- DE-A1-102007 039 004
- US-A1- 2011 004 108

## Beschreibung

Die vorliegende Erfindung betrifft eine Vorrichtung zur Steuerung des Atemgas-Druckes in Abhängigkeit von der Herzleistung.
Intelligente Schlaftherapiegeräte oder Heimbeatmungsgeräte stellen Therapieparameter selbsttätig ein auf Basis von Messparametern, die sie während der Therapie erheben. So regeln APAP-Geräte (Automatic Positive Airway Pressure) den Therapiedruck in Abhängigkeit von detektierten Obstruktionen, wie zum Beispiel dem Auftreten von Schnarchen, Flusslimitierungen oder obstruktiven Apnoen. Die automatische Detektion erfolgt auf Basis einer Analyse von Signalen, beispielsweise dem Fluss- oder Drucksignal. Automatische Bilevel-Geräte und Servoventilationsgeräte zur Behandlung zentraler oder gemischter Atemstörungen regeln den Beatmungshub insbesondere in Reaktion auf das Auftreten zentral bedingter Atemstörungen wie Hypoventilationen und Cheyne-Stokes-Atmung.
Den aktuellen Therapiealgorithmen ist gemein, dass die Einstellung des Beatmungsdrucks in Abhängigkeit des Auftretens obstruktiver Atemstörungen erfolgt, und die Einstellung des Druckhubs (inspiratorischer Druck minus exspiratorischer Druck), insbesondere in Abhängigkeit des Auftretens zentraler Atemstörungen erfolgt.
Die Herzinsuffizienz-Erkrankung kann sowohl mit obstruktiv als auch mit zentral bedingten Ereignissen verknüpft sein, oder mit beidem. Herzinsuffizienzbedingte Flüssigkeitsumlagerungen und Stauungen im Körper können zentrale und obstruktive Ereignisse hervorrufen.

Da der eingestellte Beatmungsdruck vom Auftreten obstruktiver Atem-Ereignisse abhängt, führt er im Regelfall nicht zur optimalen Druckunterstützung des Herzens bezüglich der Herzleistung (CO). Wenn keine obstruktiven Ereignisse auftreten, bleibt der Druck für die Herzleistung suboptimal. Mögliche Folgen sind eine weitere Verschlechterung der Herzleistung, Begünstigung von Ödemen, weitere Schlafbezogene Atmungsstörungen (SBAS).

Die EP2923720 A1 offenbart eine Vorrichtung zur Behandlung von Cheyne-Stokes-Atmung, periodischer Atmung und zentralen oder gemischten Apnoen mit dem Ziel, eine adaptive Druckunterstützung anzugeben, um Schwankungen beim Atemantrieb des Patienten auszugleichen, die jedoch die aktuelle natürliche Atmung des Patienten berücksichtigt.

Die EP 2 216 063 A2 offenbart eine Vorrichtung zur Erkennung von Komplikationen während der Beatmung.

Die DE 10 2007 039 004 A1 offenbart eine Vorrichtung zur Bilevel Beatmung.

Die US 2011/0004108 A1 offenbart eine Methode zur Bestimmung des cardiac output (Herzzeitvolumen). Dazu verwendet die US 2011/0004108 A1 ein Anästhesiegerät mit Ventilator und eine Vielzahl unterschiedlicher Sensoren. Eine Maßnahme die zusätzlich zu der Bestimmung des cardiac output angewand wird, ist die induzierte Änderung der alveolären Ventilation der Lunge.

Die Aufgabe ist daher, einen einfachen und kostengünstigen Aufbau für die Bestimmung der Herzleistung im Rahmen der Beatmung anzugeben, wobei die Beatmung zur Unterstützung des Herzens optimiert werden soll ohne dabei eine Beatmung zur Unterstützung von Atmungseinschränkung zu vernachlässigen.
Die Erfindung basiert auf der Erkenntnis, dass die Herzleistung durch den Überdruck in den Atemwegen beeinflusst wird.

Diese Aufgabe wird durch eine Vorrichtung mit den Merkmalen des Anspruchs 1 gelöst.
Einige Vorteile und Merkmale der vorliegenden Erfindung ergeben sich aus den Unteransprüchen. Weitere Vorteile und Merkmale sind in der allgemeinen Beschreibung und der Beschreibung der Ausführungsbeispiele angegeben.

Die erfindungsgemäße Vorrichtung zur Veränderung des Atemgas-Druckes bei der Beatmung in Abhängigkeit von einem Maß der Herzleistung umfasst eine Atemgasquelle zum Abgeben einer gesteuerten Atemgaszufuhr unter einem Überdruck zu einem Patienten, zumindest eine Sensoreinrichtung zur Ableitung eines Maßes der Herzleistung (CO) und eine Steuerung zur Vorgabe des Atemgas-Druckes in Abhängigkeit von dem Maß der Herzleistung (CO).
Ein Vorteil der Erfindung ist, dass ein Vergleichsmittel, welches beispielsweise als Teil der Steuerung ausgeführt ist, eine Veränderung der Herzleistung (CO) über die Zeit registriert und die Steuerung bei einem Abfall der Herzleistung (CO) den Druck erhöht.
Ein weiterer Vorteil der erfindungsgemäßen Vorrichtung ergibt sich dadurch, dass ein Vergleichsmittel eine Veränderung der Herzleistung (CO) über die Zeit registriert und die Steuerung bei einem Anstieg der Herzleistung (CO) den Druck erniedrigt.

Dadurch erhält der Patient eine bedarfsgerechte Druckunterstützung zur Optimierung seiner Herzleistung.

Ein weiterer Vorteil der erfindungsgemäßen Vorrichtung ergibt sich dadurch, dass ein Vergleichsmittel eine Veränderung der Herzleistung (CO) über die Zeit registriert und die Steuerung den EPAP-Druck (expiratory positive airway pressure) anpasst. Ein weiterer Vorteil der erfindungsgemäßen Vorrichtung ergibt sich dadurch, dass ein Vergleichsmittel eine Veränderung der Herzleistung (CO) über die Zeit registriert und die Steuerung den IPAP-Druck (inspiratory positive airway pressure) anpasst. Auf diese Weise kann der Patient mit seinem gewöhnten Rhythmus weiteratmen, wobei der Druck auf das Herz erhöht wird.

Ein weiterer Vorteil der erfindungsgemäßen Vorrichtung ergibt sich dadurch, dass die Sensoreinrichtung eingerichtet ist respiratorische Größen wie Druck oder Fluss zu ermitteln.

Ein weiterer Vorteil der erfindungsgemäßen Vorrichtung ergibt sich dadurch, dass die Sensoreinrichtung eingerichtet ist, respiratorische Größen wie Druck oder Fluss zu ermitteln.
Auf diese Weise können Standardsensoren auch zur Ermittlung eines Maßes der Herzleistung herangezogen werden.
Ein weiterer Vorteil der erfindungsgemäßen Vorrichtung ergibt sich dadurch, dass das Maß der Herzleistung aus dem Atemgas-Fluss abgeleitet ist unter Berücksichtigung von Atemfrequenz und/oder Atemzugs- oder Atemzeitvolumina und/oder (Atemzeiterhältnis Inspiration zu Exspiration) (I:E) und/oder abgeleiteten Atemereignisse wie Apnoen und/oder Hypopnoen und/oder Cheyne-Stokes(CS)-Atmung, sowie insbesondere deren Anzahl, Länge, Anteil, Schwere, Periodik.

Ein weiterer Vorteil der erfindungsgemäßen Vorrichtung ergibt sich dadurch, dass das Maß der Herzleistung aus dem Atemgas-Druck abgeleitet ist unter Berücksichtigung von Druckparametern und/oder Schall und/oder Schnarchen und/oder Atemwegswiderstand, sowie insbesondere deren Anzahl, Länge, Anteil, Schwere, Periodik.

Ein weiterer Vorteil der erfindungsgemäßen Vorrichtung ergibt sich dadurch, dass das Maß der Herzleistung aus einer plethysmographischen Messung abgeleitet ist unter Berücksichtigung von Pulswellenparametern und/oder Pulsfrequenz und/oder Sauerstoffsättigung und/oder Entsättigungen, sowie insbesondere deren Anzahl, Länge, Anteil, Schwere, Periodik. Ein weiterer Vorteil der erfindungsgemäßen Vorrichtung ergibt sich dadurch, dass das Maß der Herzleistung aus einer Bestimmung der transkutanen Kohlenstoffdioxid (CO2)-Konzentration und/der der Kohlenstoffdioxid (CO2)-Konzentration der Ausatemluft und/oder Daten von Bewegungssensoren abgeleitet ist, sowie insbesondere deren Anzahl, Länge, Anteil, Schwere, Periodik.

Ein weiterer Vorteil der erfindungsgemäßen Vorrichtung ergibt sich dadurch, dass das Maß der Herzleistung aus Befragungen des Patienten abgeleitet sind hinsichtlich Atemnot und/oder Kurzatmigkeit und/oder Belastbarkeit und/oder Befinden allgemein, sowie insbesondere deren Anzahl, Länge, Anteil, Schwere, Periodik.

Ein Vorteil der Erfindung ist, dass für das Maß der Herzleistung ein Akzeptanzfenster hinterlegt sind, innerhalb dessen keine Druckreaktion erfolgt.

Ein Vorteil der Erfindung ist, dass für das Maß der Herzleistung zumindest ein Schwellwert hinterlegt ist, oberhalb welchem eine Druckreaktion erfolgt.

Ein anderer Vorteil der Erfindung ist, dass die Steuerung schrittweise ein vorgegebenes Druckprofil abfährt und dabei schrittweise ein Maß der Herzleistung als Reaktion auf den vorgegebenen Druck bestimmt, zur Ermittlung eines individuell optimieren Druckes.

Das Verfahren zur Veränderung des Atemgas-Druckes bei der Beatmung erfolgt in Abhängigkeit von einem Maß der sensorisch abgeleiteten Herzleistung.
Der Begriff Herzleistung umfasst im Sinne dieser Erfindung jede qualitative oder quantitative Größe die geeignet ist die Leistungsfähigkeit des Herzens einzuschätzen. Dabei werden bevorzugt nicht invasiv erfassbare Größen verwendet, die auf Messwerten beruhen, die durch eine Herzaktion beeinflusst oder hervorgerufen werden.

Der Begriff Maß umfasst im Sinne dieser Erfindung direkt oder indirekt messbare oder ableitbare Größen der Herzleistung, sowie direkt oder indirekt messbare respiratorische Größen oder Kreislauf-Größen, die durch die Herzleistung beeinflusst werden und geeignet sind, Rückschlüsse auf die Herzleistung zu ziehen. Die Begriffe Atemgasquelle zum Abgeben einer gesteuerten Atemgaszufuhr unter einem Überdruck umfassen insbesondere eine steuerbare Gebläseeinrichtung, die an die Leitung gekoppelt ist, um die Versorgung mit Atemgas zu erzeugen und umfassen im Sinne dieser Erfindung jede steuerbare Quelle von Atemgas wie beispielsweise Elektromotoren mit Gebläserädern oder Druckgasquellen, die dieses unter veränderlicher Vorgabe über eine Leitung an ein Patienteninterface fördern.

Die Begriffe zumindest eine Sensoreinrichtung zur Ableitung eines Maßes der Herzleistung (CO) umfassen im Sinne dieser Erfindung sowohl Benutzerparameter als auch Geräteparameter. Die Benutzerparameter umfassen dabei insbesondere die zur Beschreibung der Atmung üblicherweise herangezogenen Parameter, wie beispielsweise die Atemfrequenz, das Atemminutenvolumen, das Atemzugvolumen und/oder der Atemgasdruck und/oder den Atemgasfluss. Möglich sind auch weitere Benutzerparameter, wie z.B. die Sauerstoffkonzentration, die Atemexkursion und/oder elektrografische Aktivitäten, Pulswelle, Pulsfrequenz, Kohlenstoffdioxid (CO2)-Konzentration, Sauerstoff (O2)-Sättigung oder Benutzerfragebögen.
Geräteparameter sind insbesondere Parameter, welche durch die Vorrichtung vorgegeben und/oder beeinflusst werden, wie zum Beispiel der Inspirationsfluss, der Inspirationsdruck und/oder EPAP-Druck und/oder andere Druckeigenschaften und/oder Flusseigenschaften und/oder eine Gebläsedrehzahl. Möglich sind auch Geräteparameter, welche eine Einstellung der Vorrichtung charakterisieren, wie beispielsweise Vorgaben von Druck oder Fluss oder Volumen oder Zeiten.

Im Sinne der Erfindung ist die Sensoreinrichtung geeignet Parameter aufzuzeichnen, der repräsentativ für die Herzleistung sind. Die Herzleistung kann hierbei das Herzminutenvolumen und/oder die Herzfrequenz und/oder das Schlagvolumen und/oder die Plusfrequenz und/oder der Blutfluss durch die Lunge und/oder die im Drucksignal des Atemgases erkennbare Druckwelle der pulsierenden Lungenarterie sein.
In den Zeichnungen sind Ausführungsbeispiele der Erfindung schematisch dargestellt. Es zeigen:
Fig. 1 eine perspektivische Darstellung der Vorrichtung zur Veränderung des Atemgas-Druckes bei der Beatmung in Abhängigkeit von einem Maß der Herzleistung mit angeschlossenem Beatmungsschlauch sowie oberhalb der Vorrichtung angeordnetem Zuschaltventil für die Zugabe von Sauerstoff und/oder Kohlendioxid.
Fig. 2 eine vergrößerte Darstellung der Einzelheit II in Fig. 1,
Fig. 3 eine Anordnung ähnlich zu Fig. 1 mit zusätzlicher Veranschaulichung einer Anzeigeeinrichtung.

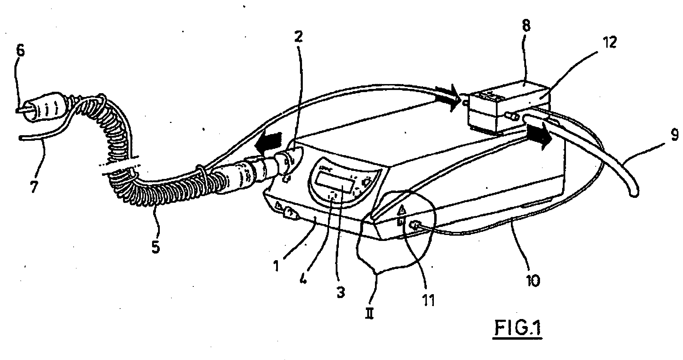

Fig. 1 zeigt eine perspektivische Darstellung der Vorrichtung (1), das mit einem Schlauchanschluss (2), einer Anzeige (3) sowie Bedienelementen (4) versehen ist. Mit dem Schlauchanschluss (2) ist ein Beatmungsschlauch (5) verbunden, durch den sich eine Druckmessleitung (6) hindurch erstreckt.

Im Bereich eines Gerätegehäuses ist in einem Geräteinnenraum eine steuerbare Atemgasquelle, beispielsweise eine Gebläseeinrichtung (19) angeordnet. Über eine Kopplung wird ein Beatmungsschlauch (5) angeschlossen. Entlang des Verbindungsschlauches kann ein zusätzlicher Druckmessschlauch verlaufen, der über einen Druckeingangsstutzen mit dem Gerätegehäuse verbindbar ist. Zur Ermöglichung einer Datenübertragung weist das Gerätegehäuse zumindest eine Schnittstelle (11) auf.

Im Bereich des Gerätegehäuses ist in einem Geräteinnenraum eine Schälldämmvorrichtung im Ansaugbereich und im Druckbereich angeordnet. Die Schälldämmvorrichtung umschließt die Steuerbare Gebläseeinrichtung zumindest bereichsweise.
Im Bereich einer dem Gerätegehäuse abgewandten Ausdehnung des Beatmungsschlauches ist ein Ausatmungselement (nicht dargestellt) angeordnet. Ebenfalls kann ein Ausatemventil verwendet werden. Als Patienteninterface (nicht dargestellt) kann eine Beatmungsmaske, die über eine Kopfhaube im Bereich eines Kopfes eines Patienten fixiert werden kann, an das Schlauchende angeschlossen werden. Im Bereich ihrer dem Beatmungsschlauch zugewandten Ausdehnung weist die Beatmungsmaske ein Kupplungselement auf.
Außenseitig am Beatmungsschlauch (5) ist eine Leitung (7) verlegt, die mit einem Zuschaltventil (8) verbunden ist. Über die Leitung können dem Atemgas Sauerstoff (O2) und/oder Kohlendioxid (CO2) zudosiert werden. Das Zuschaltventil (8) ist über eine Versorgungsleitung (9) an eine nicht dargestellte Quelle für Kohlenstoffdioxid (CO2) und/oder Sauerstoff (O2) angeschlossen. Über eine Steuerleitung (10) ist das Zuschaltventil (8) mit einem Steueranschluss (11) der Vorrichtung (1) verbunden. Der Steueranschluss (11) ist mit einem in Fig. 1 nicht dargestellten Prozessor der Vorrichtung (1) verbunden.
Unter Verwendung des Zuschaltventils (8) können einem an das Beatmungsgerät (1) angeschlossenen Patienten typischerweise bis zu 50 Liter/Minute Sauerstoff zugeleitet werden. Alternativ und oder ergänzend kann das Atemgas auf 2 % bis 5 % Kohlenstoffdioxid (CO2) angereichert werden.
Die Versorgungsleitung (9) kann an einen Sauerstoffkonzentrator, eine Kohlenstoffdioxid (CO2)-Flasche mit Druckminderer, eine Sauerstofflasche mit Druckminderer, eine Flüssigsauerstoffversorgung oder eine zentrale Gasversorgung angeschlossen sein.
Eine Steuerung des Zuschaltventils (8) von der Vorrichtung (1) aus kann über unterschiedliche Schnittstellen (11) erfolgen. Die Schnittstellen können kabelgebunden, als Infrarot-Schnittstelle oder als Bluetooth-Schnittstelle realisiert sein. Insbesondere kann die Verbindung zwischen dem Zuschaltventil (8) und der Vorrichtung (1) elektrisch, pneumatisch, optisch, mechanisch oder hinsichtlich der vorstehenden Varianten gemischt realisiert sein.
Vorteilhaft für eine Nachrüstbarkeit des Zuschaltventils (8) erweist es sich insbesondere, einen bereits vorhandenen Steueranschluss (11) zur Steuerung des Zuschaltventils (8) zu verwenden. Über diese vorhandene Schnittstelle erfolgt die Kommunikation zwischen dem Zuschaltventil (8) und dem Prozessor der Vorrichtung(1). Dazu ist ein Kabel (10) mit Stecker (13) und Sicherungsmechanismus (14, 15) mit der Schnittstelle 11 verbunden. Alternativ erfolgt die Kommunikation und Steuerung wie oben geschildert drahtlos.

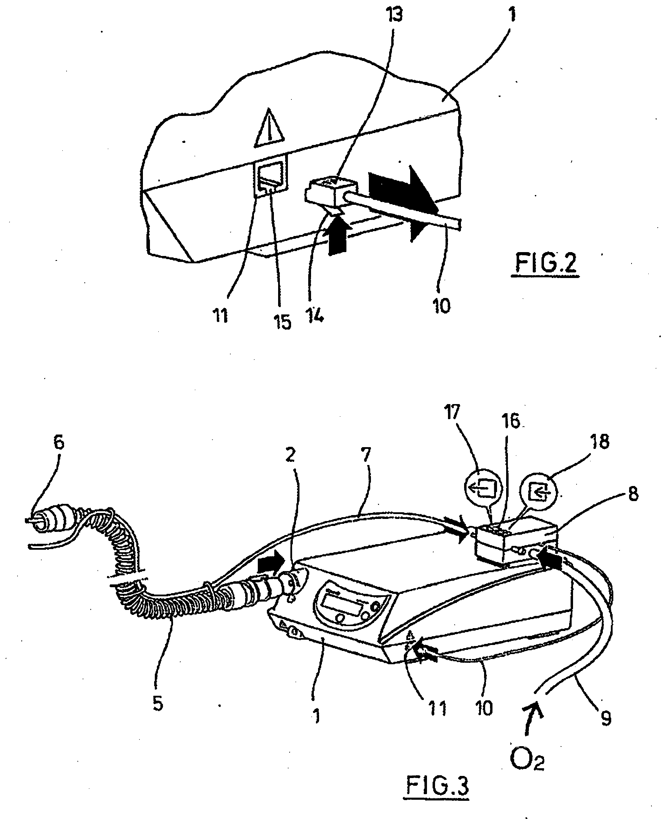

Fig. 3 zeigt eine Darstellung ähnlich zu Fig. 1 mit zusätzlicher Verdeutlichung einer Anzeige (16) im Bereich des Gehäuses (12) des Zuschaltventils (8). Die Anzeige (16) weist Statusanzeigen (17, 18) auf. Die Anzeige (16) kann optisch, akustisch oder sowohl optisch als auch akustisch realisiert sein. Die Statusanzeigen (17, 18) signalisieren die Funktion des Zuschaltventils (8). Wenn die Vorrichtung (1) ausgeschaltet ist oder nicht ordnungsgemäß arbeitet, dann schließt das Zuschaltventil (8) und die Anzeige (16) erlischt. Nach Wiedererreichen eines ordnungsgemäßen Arbeitszustandes der Vorrichtung (1) öffnet das Zuschaltventil (8) wieder.

Vorzugsweise erfasst eine Sensoreinrichtung beispielsweise den Fluss von Atemgas während Inspiration oder Exspiration. Beispielsweise kann ein Drehzahlsensor zur Erfassung der Drehzahl der Gebläseeinrichtung (19) vorgesehen sein. Möglich ist auch ein Sensor, zur Erfassung einer elektrischen Leistungsaufnahme bzw. -abgabe der Gebläseeinrichtung (19). So kann eine Druckänderung und/oder eine Änderung des Atemwegswiderstands anhand von Leistungskennzahlen der Gebläseeinrichtung (19) ermittelt werden.
Um weitere Beatmungsparameter überwachen zu können, kann die Sensoreinrichtung (20, 20') auch mit Sensoren zur Messung der Atemexkursion und/oder zur Messung einer Sauerstoffsättigung des Blutes und/oder zur Messung einer EEG-, einer EMG-, einer EOG- oder einer EKG-Aktivität ausgestattet sein.

Über eine Steuerleitung (10) ist eine Sensoreinrichtung (20') mit einem Steueranschluss (11) der Vorrichtung (1) verbunden. Der Steueranschluss (11) ist mit einem in Fig. 1 nicht dargestellten Prozessor der Vorrichtung (1) verbunden.

Unter Verwendung der Sensoreinrichtung (20') können Herzleistungswerte eines Patienten ermittelt werden. Dies ist jeder Sensor (20'), der geeignet ist einen Parameter aufzuzeichnen, der repräsentativ für die Herzleistung ist. Die Herzleistung kann hierbei das Herzminutenvolumen und/oder die Herzfrequenz und/oder das Schlagvolumen und/oder die Plusfrequenz und/oder der Blutfluss durch die Lunge und/oder die im Drucksignal des Atemgases erkennbare Druckwelle der pulsierenden Lungenarterie sein.
Die Sensoreinrichtung ist beispielsweise eingerichtet, respiratorische Größen wie Druck oder Fluss zu ermitteln.
Die Sensoreinrichtung ist beispielsweise eingerichtet, respiratorische Größen wie Druck oder Fluss zu ermitteln.
Die Vorrichtung ist beispielsweise eingerichtet, das Maß der Herzleistung aus dem Atemgas-Fluss abzuleiten unter Berücksichtigung von Atemfrequenz und/oder Atemzugs- oder Atemzeitvolumina und/oder Atemzeitverhältnis Inspiration zu Exspiration (I:E) und/oder abgeleiteten Atemereignisse wie Apnoen und/oder Hypopnoen und/oder Cheyne-Stokes (CS)-Atmung, sowie insbesondere deren Anzahl, Länge, Anteil, Schwere, Periodik.
Die Vorrichtung ist beispielsweise eingerichtet, das Maß der Herzleistung aus dem Atemgas-Druck abzuleiten unter Berücksichtigung von Druckparametern und/oder Schall und/oder Schnarchen und/oder Atemwegswiderstand, sowie insbesondere deren Anzahl, Länge, Anteil, Schwere, Periodik.
Die Vorrichtung ist beispielsweise eingerichtet, das Maß der Herzleistung aus einer plethysmographischen Messung abzuleiten unter Berücksichtigung von Pulswellenparametern und/oder Pulsfrequenz und/oder Sauerstoffsättigung und/oder Entsättigungen, sowie insbesondere deren Anzahl, Länge, Anteil, Schwere, Periodik.
Die Vorrichtung ist beispielsweise eingerichtet, das Maß der Herzleistung aus einer Bestimmung der transkutanen Kohlenstoffdioxid (CO2)-Konzentration und/der der Kohlenstoffdioxid (CO2)-Konzentration der Ausatemluft und/oder Daten von Bewegungssensoren abzuleiten, sowie insbesondere deren Anzahl, Länge, Anteil, Schwere, Periodik.
Die Vorrichtung ist beispielsweise eingerichtet, das Maß der Herzleistung aus Befragungen des Patienten abzuleiten hinsichtlich Atemnot und/oder Kurzatmigkeit und/oder Belastbarkeit und/oder Befinden allgemein, sowie insbesondere deren Anzahl, Länge, Anteil, Schwere, Periodik.

Eine Steuerung der Sensoreinrichtung (20') von der Vorrichtung (1) aus kann über unterschiedliche Schnittstellen (11) erfolgen. Die Schnittstellen können kabelgebunden, als Infrarot-Schnittstelle oder als Bluetooth-Schnittstelle realisiert sein. Insbesondere kann die Verbindung zwischen Sensoreinrichtung (20') und der Vorrichtung (1) elektrisch, pneumatisch, optisch, mechanisch oder hinsichtlich der vorstehenden Varianten gemischt realisiert sein.

Vorteilhaft für eine Nachrüstbarkeit der Sensoreinrichtung (20') erweist es sich insbesondere, einen bereits vorhandenen Steueranschluss (11) zur Steuerung der Sensoreinrichtung (20') zu verwenden. Über diese vorhandene Schnittstelle erfolgt die Kommunikation zwischen Sensoreinrichtung (20') und dem Prozessor der Vorrichtung (1). Dazu ist ein Kabel (10) mit Stecker (13) und Sicherungsmechanismus (14, 15) mit der Schnittstelle 11 verbunden. Alternativ erfolgt die Kommunikation und Steuerung wie oben geschildert drahtlos.

Eine Anzeige (16) im Bereich des Gehäuses (12) der Sensoreinrichtung (20') kann Statusanzeigen (17, 18) aufweisen. Die Anzeige (16) kann optisch, akustisch oder sowohl optisch als auch akustisch realisiert sein. Die Statusanzeigen (17, 18) signalisieren die Funktion der Sensoreinrichtung (20') Wenn die Vorrichtung (1) ausgeschaltet ist oder nicht ordnungsgemäß arbeitet, dann fährt auch die Sensoreinrichtung (20') herunter und die Anzeige (16) erlischt.
Des Weiteren weist die Vorrichtung eine Steuerung (22) zur Ansteuerung der Atemgasquelle (19) auf. Die Steuerung (22) stellt einen notwendigen Minimaldruck bereit und kompensiert Druckschwankungen, die durch die Atemtätigkeit des Benutzers bedingt sind und regelt die Leistung der Gebläseeinrichtung (19) entsprechend nach, bis ein gewünschter Beatmungsdruck anliegt. Dabei ist die Steuerung (22) vorzugsweise so ausgelegt, dass eine Druckgenauigkeit von +/- 0,3 hPa erreicht wird. Vorzugsweise regelt die Steuerung auch den Ruheluftstrom entsprechend.

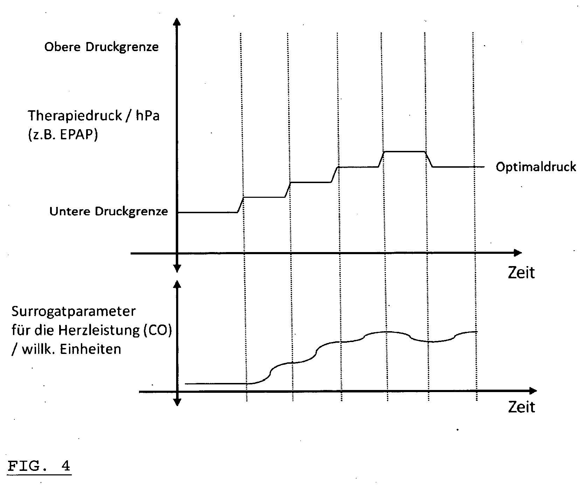

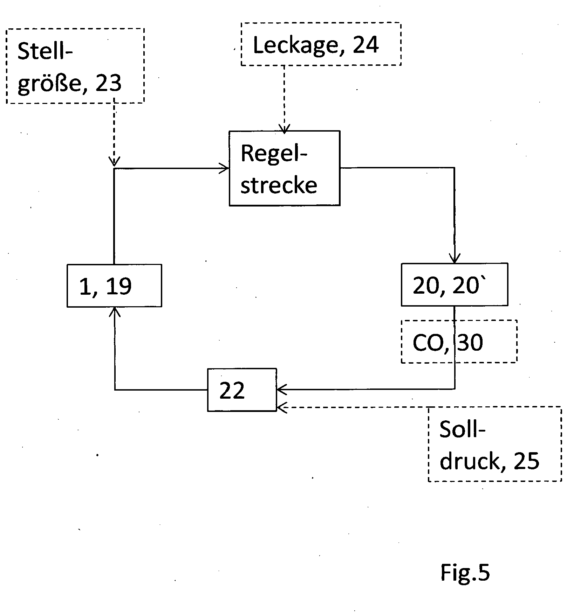

Fig. 4 zeigt die Iterative Druckänderung durch die Steuerung der Vorrichtung zur Bestimmung des Druckes anhand eines Maßes der Herzleistung. Im unteren Teil der Figur 4 ist der Verlauf des Maßes der Herzleistung im Zeitverlauf dargestellt. Im oberen Teil der Figur 4 ist der Verlauf des in Abhängigkeit des Maßes der Herzleistung von der Steuereinrichtung eingestellten Druckes (beispielsweise EPAP) im Zeitverlauf dargestellt.

Wird das Druckniveau verändert, so wird die Auswirkung auf die Herzleistung bestimmt. Kann aus der Änderung auf eine Verbesserung der Herzleistung geschlossen werden, wird das Druckniveau in die gleiche Richtung verändert. Kann aus der Änderung auf eine Verschlechterung der Herzleistung geschlossen werden, wird das Druckniveau in die Gegenrichtung verändert. Es handelt sich dabei um ein iteratives Vorgehen: geringfügige Veränderung des Druckes können zu messbaren Veränderungen mindestens einer physiologischen Größe führen z. B. aus der Atmung. Ergibt eine Bewertung dieser Veränderung eine Verbesserung oder Verschlechterung der Herzleistung so erfolgt die nächste Veränderung in dieselbe Richtung oder Gegenrichtung zur Verbesserung des Maßes der Herzleistung. Alternativ kann der Druck auch durch das Abfahren eines festgelegten Druckprofiles bestimmt werden. Parallel dazu erfolgt dann die Bestimmung des Maßes der Herzleistung. Auf diesem Wege kann die Steuerung den Druck so lange anheben wie sich das Maß der Herzleistung verbessert. Verschlechtert sich das Maß der Herzleistung so wird der Druck wieder abgesenkt. Um den individuell optimalen Druck zu finden wird solange der Druck angehoben und wieder abgesenkt und dabei das das Maß der Herzleistung bestimmt, bis ein idealer Druck gefunden ist, dieser wird anschließend fest vorgegeben.

Alternativ oder ergänzend wird zunächst ein Maß der Herzleistung bestimmt und anhand hinterlegter Werte bewertet. Alternativ oder ergänzend wird über einen Zeitraum von zumindest drei Atemzyklen ein Maß der Herzleistung bestimmt und ein Trend der Entwicklung bestimmt. Ergibt die Bewertung ein ungünstiges Maß oder einen abfallenden Trend, so wird der Druck angehoben. Um den individuell optimalen Druck zu finden wird solange der Druck angehoben und wieder abgesenkt und dabei das das Maß der Herzleistung bestimmt, bis ein idealer Druck gefunden ist, dieser wird anschließend fest vorgegeben.

Inspiratorische und exspiratorische Drücke können im Bereich 4 - 30 hPa liegen. die Druckdifferenz IPAP-EPAP liegt zwischen 0 - 25 hPa. Die Höhe der iterativen Druckanpassung 0,1 - 3 hPa pro Zeiteinheit, bevorzugt 0,5 - 2,5 hPa pro Zeiteinheit. Als Zeiteinheit dient zumindest ein Atemzyklus bis zu einem Tag. Die Zeitbasis dieser Veränderungen des Druckes kann sehr kurzfristig auf der Skala weniger Sekunden oder Minuten erfolgen, wenn beispielsweise wenn morphologische Parameter aus dem Puls- oder Flusssignal als Maß für die Herzleistung verwendet werden. Die Schritte der Anpassung des Druckes können aber auch über mehrere Tage oder Nächte erfolgen, wenn beispielsweise als Maß für die Herzleistung ist, das Auftreten von Cheyne-Stokes-Atmung verwendet wird, oder eine Abfrage der Symptome beim Patienten. Eine Kombination verschiedener Parameter auf unterschiedlichen Zeitskalen ist ebenfalls möglich.
Messgrößen können sein: Größen, die aus einer Flowmessung abgeleitet werden wie morphologische Flowparameter, Atemfrequenz, Atemzugs- oder Atemzeitvolumina, Atemzeitverhältnis Inspiration zu Exspiration (I:E), abgeleitete Atemereignisse wie Apnoen, Hypopnoen, Cheyne-Stokes (CS)-Atmung, deren Anzahl, Länge, Anteil, Schwere, Periodik, Größen, die aus einer Druckmessung abgeleitet sind wie morphologische Druckparameter, Schall, Schnarchsignalanteile, Atemwegswiderstände; Größen, die aus einer plethysmographischen Messung abgeleitet sind wie Pulswellenparameter, Herzfrequenz, Sauerstoffsättigung,Größen, die aus Befragungen des Patienten abgeleitet sind wie Symptomabfragen von Atemnot, Kurzatmigkeit, Belastbarkeit, Befinden allgemein, (...) ; Größen, die aus weiteren angeschlossenen Geräten oder Sensoren extrahiert werden wie transkutane Kohlenstoffdioxid (CO2)-Konzentration, Daten von Bewegungssensoren, (...).
Diese herzleistungsorientierte Drucksteuerung kann kombiniert werden mit einer Drucksteuerung gegen Obstruktionen, wie sie aus APAP-Geräten bekannt ist. Dabei werden die Ziele "Maximale Herzleistung" und "wenig/keine Obstruktionen" geeignet gegeneinander priorisiert, sofern die resultierenden Optimaldrücke in Widerspruch zueinander stehen. Dieser Konflikt kann beispielsweise automatisch gelöst werden durch Hinterlegen entsprechender Priorisierungsregeln im Gerät. Alternativ kann das Gerät auf den Konflikt aufmerksam machen und eine Entscheidung durch einen Bediener einholen. Die Priorisierung kann im Sinne von "Scopes" auch vorher durch den Bediener vorgegeben werden. Der resultierende Druck kann direkt dem Optimaldruck bezüglich Obstruktionsverhinderung oder bezüglich Herzleistung entsprechen, oder einem Kompromiss zwischen beiden darstellen.
Zur Therapie von Cheyne-Stokes (CS)-Atmung ist eine Kombination mit antizyklischer Druckunterstützung möglich.
Für die automatische Einstellung der inspiratorischen und exspiratorischen Drücke sowie der Differenzen zwischen ihnen können Grenzen vorgegeben werden. Der Betrag von Druckanpassungen muss nicht konstant sein, sondern kann abhängig vom Abstand des aktuellen Druckwerts von den Grenzen oder abhängig vom Absolutwert aktueller Druckeinstellungen variabel sein.
Je nach eingestelltem Druckprofil kann das Druckniveau direkt dem CPAP entsprechen (bei CPAP und APAP-Modus), dem EPAP (bei Bilevel-Beatmung), dem EEPAP (bei TriLevel-Beatmung), einem auf den Patienten adaptierten exspiratorischem Beatmungsdruck im Falle individuell adaptierender Beatmungsmodi, oder einem mittleren Beatmungsdruck in all diesen Modi.

Orientiert sich die Einstellung der Therapiedrücke und Druckhübe nicht oder nicht ausschließlich an dem Auftreten von Atmungsstörungen, sondern auch an der Herzleistung, kann bezüglich der Herzinsuffizienz selbst und nicht nur in ihren Symptomen eine Verbesserung beim Patienten erreicht werden. So kann die Herzleistung gesteigert werden, und so die Cheyne-Stokes (CS)-Atmung reduziert oder verhindert werden, und/oder weitere Herzinsuffizienzsymptome wie Lungen- und weitere Ödeme können reduziert oder verhindert werden.
Von einer solchen Therapie profitieren nicht nur Patienten, die schlafbezogene Atmungsstörungen zeigen, sondern alle Herzinsuffizienzpatienten, deren Herzleistung durch einen positiven Atemwegsdruck gesteigert werden kann. Dabei ist die Anwendung nicht nur auf die Nacht beschränkt, sondern kann insbesondere in Akutphasen wie Dekompensationen oder Rekompensationen auch tagsüber eingesetzt werden.

## Patentansprüche

1. Vorrichtung zur Veränderung des Atemgas-Druckes bei der Beatmung in Abhängigkeit von einem Maß der Herzleistung (30), mit einer Atemgasquelle (19) zum Abgeben einer gesteuerten Atemgaszufuhr unter einem Überdruck zu einem Patienten, zumindest einer Sensoreinrichtung (20, 20') zur Ableitung eines Maßes der Herzleistung (30) einer Steuerung (22) zur Vorgabe des Atemgas-Druckes in Abhängigkeit von dem Maß der Herzleistung (30) **dadurch gekennzeichnet, dass** das Maß der Herzleistung, unter Verwendung der Sensoreinrichtung (20, 20), aus einer plethysmographischen Messung (20, 20') abgeleitet ist unter Berücksichtigung von Pulswellenparametern und/oder Pulsfrequenz und/oder Sauerstoffsättigung und/oder Entsättigungen, sowie insbesondere deren Anzahl, Länge, Anteil, Schwere, Periodik, wobei die Sensoreinrichtung eingerichtet ist, respiratorische Größen wie Druck oder Fluss zu ermitteln um ein Maß der Atmungseinschränkung zu bestimmen, wobei in das Maß eingehen können Atemfrequenz und/oder Atemzugs- oder Atemzeitvolumina und/oder Atemzeitverhältnis Inspiration zu Exspiration (I:E) und/oder abgeleiteten Atemereignisse wie Apnoen und/oder Hypopnoen und/oder Schall und/oder Cheyne-Stokes(CS)-Atmung und/oder Obstruktionen sowie insbesondere deren Anzahl, Länge, Anteil, Schwere, Periodik und wobei die Vorrichtung zur Veränderung des Atemgas-Druckes mit zumindest einer Sensoreinrichtung zur Ableitung eines Maßes der Atmungseinschränkung und einem Prozessor zur Berechnung zumindest eines Atemgas-Druckes unter Berücksichtigung von dem Maß der Herzleistung (30) und zur Berechnung zumindest eines Atemgas-Druckes unter Berücksichtigung von dem Maß der Atmungseinschränkung und einer Priorisierungsschaltung ausgestattet ist, und die Priorisierungsschaltung die vom Prozessor berechneten Drücke anhand hinterlegter Regeln und/oder anhand einer Anwenderauswahl priorisiert, wobei die Steuerung (22) zur Vorgabe zumindest eines Atemgas-Druckes unter Berücksichtigung von dem Maß der Herzleistung (30) und unter Berücksichtigung von dem Maß der Atmungseinschränkung und unter Berücksichtigung der Priorisierung ausgebildet ist, wobei die Steuerung (22) die Vorgabe zumindest eines Atemgas-Druckes mit einer kurzen Reaktionszeit, die zwischen einer Anpassung pro Atemzug und einer Anpassung pro Therapietag liegt, umsetzt.

2. Vorrichtung nach Anspruch 1 **dadurch gekennzeichnet, dass** die Steuerung (22) ein Vergleichsmittel (22') aufweist, welches eine Veränderung der Herzleistung über die Zeit registriert und die Steuerung (22) bei einem Abfall der Herzleistung (30) den Druck erhöht.

3. Vorrichtung nach Anspruch 1 oder 2 **dadurch gekennzeichnet, dass** die Steuerung (22) ein Vergleichsmittel (22') aufweist, welches eine Veränderung der Herzleistung über die Zeit registriert und die Steuerung (22) bei einem Anstieg der Herzleistung den Druck erniedrigt.

4. Vorrichtung nach zumindest einem der vorhergehenden Ansprüche **dadurch gekennzeichnet, dass** die Steuerung (22) ein Vergleichsmittel (22') aufweist, welches eine Veränderung der Herzleistung (30) über die Zeit registriert und die Steuerung (22) den IPAP-Druck (inspiratory positive airway pressure) (23) anpasst.

5. Vorrichtung nach zumindest einem der vorhergehenden Ansprüche **dadurch gekennzeichnet, dass** die Sensoreinrichtung (20, 20') eingerichtet ist respiratorische Größen wie Druck oder Fluss zu ermitteln.

6. Vorrichtung nach zumindest einem der vorhergehenden Ansprüche **dadurch gekennzeichnet, dass** das Maß der Herzleistung (30), unter Verwendung der Sensoreinrichtung (20, 20'), aus dem Atemgas- Fluss abgeleitet ist unter Berücksichtigung von Atemfrequenz und/oder Atemzugs- oder Atemzeitvolumina und/oder Atemzeitverhältnis Inspiration zu Exspiration (I:E)und/oder abgeleiteten Atemereignisse wie Apnoen und/oder Hypopnoen und/oder Cheyne-Stokes(CS)-Atmung, sowie insbesondere deren Anzahl, Länge, Anteil, Schwere, Periodik.

7. Vorrichtung nach zumindest einem der vorhergehenden Ansprüche **dadurch gekennzeichnet, dass** das Maß der Herzleistung (30), unter Verwendung der Sensoreinrichtung (20, 20'), aus dem Atemgas-Druck abgeleitet ist unter Berücksichtigung von Druckparametern und/oder Schall und/oder Schnarchen und/oder Atemwegswiderstand, sowie insbesondere deren Anzahl, Länge, Anteil, Schwere, Periodik.

8. Vorrichtung nach zumindest einem der vorhergehenden Ansprüche **dadurch gekennzeichnet, dass** das Maß der Herzleistung, unter Verwendung der Sensoreinrichtung (20, 20') , aus einer Bestimmung der transkutanen Kohlenstoffdioxid (C02)-Konzentration und/der der Kohlenstoffdioxid (C02)-Konzentration der Ausatemluft und/oder Daten von Bewegungssensoren (20, 20') abgeleitet ist, sowie insbesondere deren Anzahl, Länge, Anteil, Schwere, Periodik.

9. Vorrichtung nach zumindest einem der vorhergehenden Ansprüche **dadurch gekennzeichnet, dass** das Maß der Herzleistung, unter Verwendung der Sensoreinrichtung (20, 20') aus einer nichtinvasiven Messung (20, 20') abgeleitet ist, bei der ein Körperteil mit Licht bestimmter Wellenlängen durchstrahlt wird und ein Bildsensor das durch den Körper veränderte Licht detektiert und daraus, anhand hinterlegter Daten und Vergleichsregeln das Maß der Herzleistung bestimmt.

10. Vorrichtung nach zumindest einem der vorhergehenden Ansprüche **dadurch gekennzeichnet, dass** für das Maß der Herzleistung (30), in der Steuerung (22) ein Akzeptanzfenster hinterlegt ist, innerhalb dessen keine Druckreaktion erfolgt.

11. Vorrichtung nach zumindest einem der vorhergehenden Ansprüche **dadurch gekennzeichnet, dass** für das Maß der Herzleistung, in der Steuerung (22) zumindest ein Schwellwert hinterlegt ist, oberhalb oder unterhalb welchem eine Druckreaktion erfolgt.

12. Vorrichtung nach zumindest einem der vorhergehenden Ansprüche **dadurch gekennzeichnet, dass** die Steuerung (22) schrittweise ein vorgegebenes Druckprofil abfährt und dabei, unter Verwendung der Sensoreinrichtung (20, 20') , schrittweise ein Maß der Herzleistung als Reaktion auf den vorgegebenen Druck bestimmt, zur Ermittlung eines individuell optimieren Druckes.

## Claims

1. A device for varying the breathing gas pressure during ventilation depending on a measure of the cardiac output (30), having a breathing gas source (19) for delivering a controlled breathing gas supply at an excess pressure to a patient, at least one sensor apparatus (20, 20') for deducing a measure of the cardiac output (30) of a control system (22) in order to specify the breathing gas pressure depending on the measure of the cardiac output (30), **characterized in that** the measure of the cardiac output, using the sensor apparatus (20, 20), is deduced from a plethysmographic measurement (20, 20'), taking account of pulse wave parameters and/or pulse rate and/or oxygen saturation and/or desaturations, as well as in particular the number, length, proportion, severity and periodicity thereof, wherein the sensor apparatus is designed to establish respiratory variables such as pressure or flow in order to determine a measure of the breathing restriction, wherein respiratory frequency and/or tidal volume or minute ventilation and/or inspiration to expiration (I:E) breathing time ratio and/or deduced respiratory events such as apneas and/or hypopneas and/or sound and/or Cheyne-Stokes (CS) respiration and/or obstructions as well as in particular the number, length, proportion, severity and periodicity thereof can be included in the measure, and wherein the device for varying the breathing gas pressure is equipped with at least one sensor apparatus for deducing a measure of the cardiac output (30) and at least one sensor apparatus for deducing a measure of the breathing restriction and a processor for calculating at least one breathing gas pressure, taking account of the measure of the cardiac output (30), and for calculating at least one breathing gas pressure, taking account of the measure of the breathing restriction, and a prioritization circuit, and the prioritization circuit prioritizes the pressures calculated by the processor on the basis of stored rules and/or on the basis of a user selection, wherein the control system (22) is configured to specify at least one breathing gas pressure, taking account of the measure of the cardiac output (30) and taking account of the measure of the breathing restriction and taking account of the prioritization, wherein the control system (22) implements the specification of at least one breathing gas pressure with a short response time which lies between an adjustment per breath and an adjustment per therapy day.

2. The device according to Claim 1, **characterized in that** the control system (22) has a comparison means (22') which records a variation of the cardiac output over time and the control system (22) increases the pressure in the event of a drop in the cardiac output (30).

3. The device according to Claim 1 or 2, **characterized in that** the control system (22) has a comparison means (22') which records a variation of the cardiac output over time and the control system (22) lowers the pressure in the event of an increase in the cardiac output.

4. The device according to at least one of the preceding claims, **characterized in that** the control system (22) has a comparison means (22') which records a variation of the cardiac output (30) over time and the control system (22) adjusts the IPAP (inspiratory positive airway pressure) (23).

5. The device according to at least one of the preceding claims, **characterized in that** the sensor apparatus (20, 20') is designed to establish respiratory variables such as pressure or flow.

6. The device according to at least one of the preceding claims, **characterized in that** the measure of the cardiac output (30) is deduced, using the sensor apparatus (20, 20'), from the breathing gas flow, taking account of the respiratory frequency and/or tidal volume or minute ventilation and/or inspiration to expiration (I:E) breathing time ratio and/or deduced respiratory events such as apneas and/or hypopneas and/or Cheyne-Stokes (CS) respiration, as well as in particular the number, length, proportion, severity and periodicity thereof.

7. The device according to at least one of the preceding claims, **characterized in that** the measure of the cardiac output (30) is deduced, using the sensor apparatus (20, 20'), from the breathing gas pressure, taking account of pressure parameters and/or sound and/or snoring and/or airway resistance, as well as in particular the number, length, proportion, severity and periodicity thereof.

8. The device according to at least one of the preceding claims, **characterized in that** the measure of the cardiac output is deduced, using the sensor apparatus (20, 20'), from a determination of the transcutaneous carbon dioxide (CO2) concentration and/or of the carbon dioxide (CO2) concentration of the exhaled air and/or data from movement sensors (20, 20'), as well as in particular the number, length, proportion, severity and periodicity thereof.

9. The device according to at least one of the preceding claims, **characterized in that** the measure of the cardiac output is deduced, using the sensor apparatus (20, 20'), from a non-invasive measurement (20, 20'), in which a part of the body is irradiated with light of determined wavelengths and an image sensor detects the light varied by the body and determines therefrom, on the basis of stored data and comparison rules, the measure of the cardiac output.

10. The device according to at least one of the preceding claims, **characterized in that** an acceptance window is stored in the control system (22) for the measure of the cardiac output (30), within which acceptance window no pressure response takes place.

11. The device according to at least one of the preceding claims, **characterized in that** at least one threshold value is stored in the control system (22) for the measure of the cardiac output, above or below which threshold value a pressure response takes place.

12. The device according to at least one of the preceding claims, **characterized in that** the control system (22) gradually starts off a specified pressure profile and, using the sensor apparatus (20, 20), gradually determines a measure of the cardiac output as a response to the specified pressure in order to establish an individually optimized pressure.

## Revendications

1. Dispositif destiné à modifier la pression de gaz respiratoire lors de la respiration artificielle en fonction d'une dimension de la performance cardiaque (30), avec une source de gaz respiratoire (19) servant à fournir un apport contrôlé de gaz respiratoire en surpression à un patient, avec au moins un dispositif détecteur (20, 20') servant à déduire une dimension de la performance cardiaque (30) d'une commande (22) pour prédéfinir la pression de gaz respiratoire en fonction de la dimension de la performance cardiaque (30), **caractérisé en ce que** la dimension de la performance cardiaque est dérivée d'une mesure pléthysmographique (20, 20) moyennant l'emploi du dispositif détecteur (20, 20') compte tenu des paramètres de l'onde pulsatoire et/ou de la fréquence du pouls et/ou de la saturation en oxygène et/ou de désaturations ainsi que notamment de leur nombre, de leur longueur, de leur proportion, de leur gravité, de leur périodicité et dans lequel le dispositif détecteur est mis en place pour évaluer des grandeurs respiratoires telles que la pression ou le débit afin d'établir une dimension de l'insuffisance respiratoire, dans lequel la dimension peut prendre en considération la fréquence respiratoire et/ou le volume respiratoire courant ou le débit ventilatoire par minute et/ou le rapport des durées inspiratoires et expiratoires (I:E) et/ou les événements respiratoires dérivés tels que les apnées, et/ou les hypopnées et/ou le bruit respiratoire et/ou la dyspnée de Cheyne-Stokes (CS) et/ou des obstructions ainsi que notamment leur nombre, leur longueur, leur proportion, leur gravité, leur périodicité et dans lequel le dispositif destiné à modifier la pression de gaz respiratoire est pourvu d'au moins un dispositif détecteur servant à déduire une dimension de la performance cardiaque (30) et d'au moins un dispositif détecteur servant à déduire une dimension de l'insuffisance respiratoire et d'un processeur servant à calculer au moins une pression de gaz respiratoire compte tenu de la dimension de la performance cardiaque (30) et servant à calculer au moins une pression de gaz respiratoire compte tenu de la dimension de l'insuffisance respiratoire et d'un circuit de priorisation, et le circuit de priorisation priorise les pressions calculées par le processeur à l'aide des règles enregistrées et/ou à l'aide d'une sélection de l'utilisateur, dans lequel la commande (22) est conçue pour prédéfinir au moins une pression de gaz respiratoire compte tenu de la dimension de la performance cardiaque (30) et compte tenu de la dimension de l'insuffisance respiratoire et compte tenu de la priorisation, dans lequel la commande (22) met en œuvre la valeur prédéfinie d'au moins une pression de gaz respiratoire avec un temps de réaction bref, qui se situe entre un ajustement par cycle respiratoire et un ajustement par jour de traitement.

2. Dispositif selon la revendication 1 **caractérisé en ce que** la commande (22) présente un moyen de comparaison (22'), lequel enregistre une variation de la performance cardiaque au fil du temps, et que la commande (22) augmente la pression en présence d'une chute de la performance cardiaque (30).

3. Dispositif selon la revendication 1 ou 2 **caractérisé en ce que** la commande (22) présente un moyen de comparaison (22'), lequel enregistre une variation de la performance cardiaque au fil du temps, et que la commande (22) diminue la pression en présence d'une hausse de la performance cardiaque.

4. Dispositif selon au moins une des revendications précédentes, **caractérisé en ce que** la commande (22) présente un moyen de comparaison (22'), lequel enregistre une variation de la performance cardiaque (30) au fil du temps, et que la commande (22) ajuste la pression IPAP (inspiratory positive airway pressure ou *pression inspiration positive dans la voie aérienne*) (23).

5. Dispositif selon au moins une des revendications précédentes, **caractérisé en ce que** le dispositif détecteur (20, 20') est mis en place pour évaluer des grandeurs respiratoires telles que la pression ou le débit.

6. Dispositif selon au moins une des revendications précédentes, **caractérisé en ce que** la dimension de la performance cardiaque (30) est dérivée du débit du gaz respiratoire moyennant l'emploi du dispositif détecteur (20, 20') compte tenu de la fréquence respiratoire et/ou du volume respiratoire courant ou du débit ventilatoire par minute et/ou du rapport des durées inspiratoires et expiratoires (I:E) et/ou des événements respiratoires dérivés tels que les apnées, et/ou les hypopnées et/ou la dyspnée de Cheyne-Stokes (CS), ainsi que notamment de leur nombre, de leur longueur, de leur proportion, de leur gravité, de leur périodicité.

7. Dispositif selon au moins une des revendications précédentes, **caractérisé en ce que** la dimension de la performance cardiaque (30) est dérivée de la pression du gaz respiratoire moyennant l'emploi du dispositif détecteur (20, 20') compte tenu des paramètres de pression et/ou du bruit respiratoire et/ou du ronflement et/ou de la résistance des voies aériennes, ainsi que notamment de leur nombre, de leur longueur, de leur proportion, de leur gravité, de leur périodicité.

8. Dispositif selon au moins une des revendications précédentes, **caractérisé en ce que** la dimension de la performance cardiaque est dérivée moyennant l'emploi du dispositif détecteur (20, 20') de la détermination de la concentration de dioxyde de carbone (C02) transcutané et/ou de la concentration de dioxyde de carbone (C02) de l'air expiré et/ou des données des détecteurs de mouvement (20, 20'), ainsi que notamment de leur nombre, de leur longueur, de leur proportion, de leur gravité, de leur périodicité.

9. Dispositif selon au moins une des revendications précédentes, **caractérisé en ce que** la dimension de la performance cardiaque est dérivée d'une mesure non effractive (20, 20') moyennant l'emploi du dispositif détecteur (20, 20'), lors de laquelle une partie du corps est exposée à une lumière traversante d'une certaine longueur d'onde et un capteur d'image détecte la lumière modifiée à travers le corps et en détermine la dimension de la performance cardiaque à l'aide de données et de règles de comparaison enregistrées.

10. Dispositif selon au moins une des revendications précédentes, **caractérisé en ce qu'**une fenêtre d'acceptation de la dimension de la performance cardiaque (30) est enregistrée dans la commande (22), à l'intérieur de laquelle aucune réaction de pression ne se produit.

11. Dispositif selon au moins une des revendications précédentes, **caractérisé en ce qu'**au moins une valeur seuil de la dimension de la performance cardiaque (30) est enregistrée dans la commande (22), au-dessus ou en dessous de laquelle une réaction de pression se produit.

12. Dispositif selon au moins une des revendications précédentes, **caractérisé en ce que** la commande (22) suit progressivement un profil de pression spécifié et détermine alors progressivement une dimension de la performance cardiaque en réaction à la pression spécifiée moyennant l'emploi du dispositif détecteur (20, 20') servant à évaluer une pression optimisée individuellement.
